# EUROPÄISCHE PATENTSCHRIFT

(11) **EP 2 601 341 B1**
(45) Veröffentlichungstag und Bekanntmachung des Hinweises auf die Patenterteilung: **21.02.2018**
(21) Anmeldenummer: 11738688.8
(22) Anmeldetag: 20.07.2011
(51) Int. Cl.: D06M 11/70, D06M 13/07, D06M 13/188, D06M 13/192, A61K 8/04, A61L 9/00, A61L 9/01, C11D 1/66, C11D 3/00, C11D 3/50, C11D 3/10, C11D 3/04, C11D 3/20, C11D 3/22

(54) **VERWENDUNG EINES TEXTILBEHANDLUNGSMITTELS ZUR ENTFERNUNG VON DEODORANT-FLECKEN**
USE OF A TEXTILE TREATMENT COMPOSITON FOR REMOVAL OF DEODORANT STAINS
UTILISATION D'UN AGENT DE TRAITEMENT DE TEXTILES DESTINÉ À ENLEVER LES TACHES DE DÉODORANT

(30) Priorität: 03.08.2010 DE 102010038829
(43) Veröffentlichungstag der Anmeldung: 12.06.2013
(73) Patentinhaber: Henkel AG & Co. KGaA, 40589 Düsseldorf (DE)
(72) Erfinder: SIEBEN, Fabian, 51379 Leverkusen (DE); STEHR, Regina, 41468 Neuss (DE); DREJA, Michael, 41469 Neuss (DE)
(86) Internationale Anmeldenummer: PCT/EP2011/062405
(87) Internationale Veröffentlichungsnummer: WO 2012/016829

(56) Entgegenhaltungen:
- EP-A1- 1 319 394
- DE-A1-102009 001 559

## Beschreibung

Die Erfindung betrifft die Verwendung eines Textilbehandlungsmittels, umfassend eine desodorierende Verbindung..

Flecken von Deodorants auf Textilien sind bekanntermaßen schwierig zu entfernende Anschmutzungen. Diese Flecken entstehen, wenn das Deodorant direkt auf das Textil gelangt, aber insbesondere auch wenn beim Tragen, die Inhaltsstoffe des Deodorants, insbesondere die Aluminiumsalze, zusammen mit Schweiß auf das Textil übertragen werden.

Durch den alkalischen pH-Wert der Waschlauge beim anschließenden Waschen der Textilien werden die Aluminiumsalze auf dem Textil ausgefällt und auf dem Gewebe fixiert, was sich durch Verkrustungen, Verfärbungen oder Geruchsbildung bemerkbar macht. Die Geruchsbildung wird verstärkt, wenn das gewaschene Textil an den Stellen mit den fixierten Aluminiumsalzen Wärme und Feuchtigkeit ausgesetzt wird, beispielsweise beim Bügeln oder beim Tragen durch die Körperwärme und neuen Schweiß.

Die Verfärbungen resultieren aus Fetten, die im Schweiß enthalten sind und auf das Textil übertragen werden, sowie aus Fett- oder Öl-haltigen Inhaltsstoffen des Deodorants.

Aus der DE 10043118 A1 ist ein Waschmittel bekannt, welches eine desodorierende Verbindung enthält und der Bildung von Schlechtgerüchen bei damit behandelten Textilien entgegen wirkt.

EP 1319394 A1 offenbart eine Zubereitung mit desodorierender Wirkung enthaltend das Zinksalz der Ricinolsäure, einen Lösungsvermittler, organische und anorganische Säuren.

DE 102009001559 A1 beschreibt ein Reinigungsmittel für harte Oberflächen, das eine Kombination aus verschiedenen anorganischen und organischen Säuren sowie mindestens ein nichtionisches Tensid enthält.

Eine Aufgabe dieser Erfindung war die Bereitstellung eines Textilbehandlungsmittels, welches in der Lage ist, die unerwünschten Effekte, wie Verkrustungen, Verfärbungen oder Geruchsbildung, die aus dem Kontakt von Deodorants und Textilien beim Waschen und/oder Tragen von Textilien entstehen, zu mindern.

Diese Aufgabe wird gelöst durch die Verwendung eines Textilbehandlungsmittels mit einem pH-Wert im Bereich von 1,5 bis 4,5, umfassend
a) eine organische Säure,
b) eine anorganische Säure,
c) ein nichtionisches Tensid und
d) eine desodorierende Verbindung
beim Waschen, Reinigen und/oder Konditionieren von textilen Flächengebilden.

Ein säurehaltiges Textilbehandlungsmittel ist in der Lage, die Verkrustungen aus Schweiß und Aluminiumsalzen zu lösen bzw. anzulösen. Dadurch kann die desodorierende Verbindung in die Verkrustungen und/oder das Gewebe eindringen und gezielt gegen Schlechtgerüche wirken. Bei Textilbehandlungsmitteln ohne Säure können die desodorierende Verbindungen nur oberflächlich wirken, da sie die hartnäckigen Verkrustungen nicht durchdringen können. Das nichtionische Tensid dient neben seiner reinigenden und/oder fettlösenden Wirkung auch als Lösungsvermittler für die Schlechtgerüche, so dass diese im Waschprozess über die Waschflotte besser abtransportiert werden können.

Es ist bevorzugt, dass die organische Säure ausgewählt ist aus der Gruppe bestehend aus Citronensäure, Ameisensäure, Essigsäure, Weinsäure, Äpfelsäure, Oxalsäure, Milchsäure und Mischungen daraus. Es ist insbesondere bevorzugt, dass die organische Säure Citronensäure und/oder Ameisensäure umfasst.

Ferner ist bevorzugt, dass die anorganische Säure ausgewählt ist aus der Gruppe bestehend aus Phosphorsäure, Salzsäure und Mischungen daraus.

In einer weiteren bevorzugten Ausführungsform ist die desodorierende Verbindung ausgewählt aus der Gruppe bestehend aus Zinkricinoleat, Zinkabietat, Cyclodextrin, ätherischen Ölen, Triethylenglycol, Natriumhydrogencarbonat und Mischungen daraus. Diese Verbindungen sind bekannte und effektive desodorierende Verbindungen.

In einer besonders bevorzugten Ausführungsform enthält das Textilbehandlungsmittel zusätzlich eine flüssige, hydrophobe Verbindung. Durch Zusatz einer flüssigen, hydrophoben Verbindung können die hauptsächlich durch Fette und/oder Öle verursachten Verfärbungen entfernt werden.

Es ist bevorzugt, dass die flüssige, hydrophobe Verbindung ausgewählt ist aus der Gruppe, umfassend Waschbenzin, C₁₁₋₁₃-Isoparaffin, C₁₆₋₂₀-Isoparaffin, C₈₋₁₃-Isoparaffin, C₉₋₁₂-Isoparaffin, Limonen, Dioctylether und Mischungen daraus. Diese Verbindungen, insbesondere Limonen und Dioctylether, weisen einen hohen Spreitwert auf und penetrieren so gut in die textilen Flächengebilde und in die darauf befindlichen Fett- und Öl-haltigen Anschmutzungen. All diese hydrophoben Verbindungen sind bei Raumtemperatur (23°C) flüssig. Aufgrund ihres niedrigen Molekulargewichts sind diese Verbindungen leicht flüchtig und können so problemlos und rückstandsfrei von den textilen Flächengebilden entfernt werden. Außerdem sind diese Verbindungen entweder geruchsneutral oder weisen sogar einen angenehmen Geruch auf.
Das Textilbehandlungsmittel weist einen pH-Wert im Bereich von 1,5 bis 4,5 auf. Durch den stark sauren pH-Wert des Textilbehandlungsmittels können die Aluminiumsalz-haltigen Verkrustungen gelöst bzw. angelöst werden.

Im Folgenden soll die Erfindung, unter anderem anhand von Beispielen, eingehender erläutert werden.

Als einen essentiellen Bestandteil enthält das Textilbehandlungsmittel eine organische Säure. Die organische Säure kann Citronensäure, Ameisensäure, Essigsäure, Weinsäure, Äpfelsäure, Oxalsäure, Milchsäure oder Mischungen daraus umfassen. Es ist insbesondere bevorzugt, dass die organische Säure Citronensäure umfasst. Citronensäure ist geruchsneutral, preiswert und weist wasserenthärtende Eigenschaften auf. In einer besonders bevorzugten Ausführungsform enthält das Textilbehandlungsmittel Citronensäure und Ameisensäure.

Die Menge an organischer Säure beträgt vorzugsweise zwischen 5 und 30 Gew.-% und mehr bevorzugt zwischen 10 und 20 Gew.-%, jeweils bezogen auf das gesamte Textilbehandlungsmittel.

Als zweiten essentiellen Bestandteil enthält das Textilbehandlungsmittel eine anorganische Säure. Die anorganische Säure umfasst bevorzugt Phosphorsäure, Salzsäure oder Mischungen daraus. Die anorganische Säure wird insbesondere zugesetzt, damit das Textilbehandlungsmittel einen sauren pH-Wert im Bereich von 1,5 bis 4,5 aufweist.

Die Menge an anorganischer Säure beträgt vorzugsweise zwischen 0,5 und 10 Gew.-% und mehr bevorzugt zwischen 1 und 5 Gew.-%, jeweils bezogen auf das gesamte Textilbehandlungsmittel.

Als dritten essentiellen Bestandteil enthält das Textilbehandlungsmittel ein nichtionisches Tensid.

Geeignete nichtionische Tenside umfassen alkoxylierte Fettalkohole, alkoxylierte Fettsäurealkylester, Fettsäureamide, Alkylpolyglucoside, alkoxylierte Fettsäureamide, Polyhydroxyfettsäureamide, N-Methylglucamide, Alkylphenolpolyglycolether und Mischungen daraus.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO, 4 EO oder 7 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Geeignet sind ferner auch eine Mischung aus einem (stärker) verzweigten ethoxylierten Fettalkohol und einem unverzweigten ethoxylierten Fettalkohol, wie beispielsweise eine Mischung aus einem C₁₆₋₁₈-Fettalkohol mit 7 EO und 2-Propylheptanol mit 7 EO. Insbesondere bevorzugt enthält das Textilbehandlungsmittel einen C₁₂₋₁₈-Fettalkohol mit 7 EO, einen C₁₂₋₁₄-Fettalkohole mit 3 EO, einen C₁₂₋₁₄-Fettalkohole mit 2 EO, einen C₁₂₋₁₆-Fettalkohole mit 7 EO oder einen C₁₃₋₁₉-Oxoalkohol mit 5 EO als nichtionisches Tensid.

Bevorzugte Aminoxide sind beispielsweise N-Kokosalkyl-N,N-dimethylaminoxid, N-Talgalkyl-N,N-dihydroxyethylaminoxid, Myristylcetyldimethylaminoxid oder Lauryldimethylaminoxid.

Außerdem können als nichtionische Tenside auch Alkylglykoside der allgemeinen Formel RO(G)ₓ eingesetzt werden, in der R einen primären geradkettigen oder methylverzweigten, insbesondere in 2-Stellung methylverzweigten aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Die Menge an nichtionischem Tensid beträgt vorzugsweise zwischen 0,5 und 20 Gew.-% und mehr bevorzugt zwischen 1 und 15 Gew.-%, jeweils bezogen auf das gesamte Textilbehandlungsmittel.

Das Textilbehandlungsmittel enthält als vierten essentiellen Bestandteil eine desodorierende Verbindung. Die desodorierende Verbindung umfasst vorzugsweise Zinkricinoleat, Zinkabietat, Cyclodextrin, ätherische Öle, Triethylenglycol, Natriumhydrogencarbonat oder Mischungen daraus, wobei Zinkricinoleat bevorzugt als desodorierende Verbindung eingesetzt wird. Die Wirkung von Zinkricinoleat beruht auf Geruchslöschung, dass heißt chemischer Bindung der riechenden Substanzen, so dass diese sensorisch nicht mehr wahrnehmbar sind, und unterscheidet sich damit im Wirkungsprinzip von anderen desodorierenden Systemen wie den geruchsüberdeckenden Verbindungen oder den geruchs(stoff)zerstörenden Verbindungen.

Die Menge an desodorierender Verbindung beträgt vorzugsweise zwischen 0,01 und 5 Gew.-% und mehr bevorzugt zwischen 0,1 und 2 Gew.-%, jeweils bezogen auf das gesamte Textilbehandlungsmittel.

In einer besonders bevorzugten Ausführungsform der Erfindung enthält das Textilbehandlungsmittel zusätzlich eine flüssige, hydrophobe Verbindung. "Flüssig" bedeutet im Zusammenhang mit dieser Erfindung, dass die eingesetzte hydrophobe Verbindung bei 23°C einen flüssigen Aggregatzustand aufweist. Die flüssige, hydrophobe Verbindung weist vorzugsweise ein Molekulargewicht im Bereich von 100 bis 600 auf. Flüssige, hydrophobe Verbindungen mit einem Molekulargewicht von 100 bis 600 sind leicht flüchtig und verbleiben nicht auf den damit behandelten textilen Flächengebilden. Somit wird das Problem einer Anreicherung der flüssigen hydrophoben Verbindung auf den textilen Flächengebilden vermieden. Es ist deshalb besonders bevorzugt, wenn das Molekulargewicht der eingesetzten hydrophoben Verbindung im Bereich von 100 bis 300 liegt.

Geeignete flüssige, hydrophobe Verbindungen umfassen Waschbenzin, C₁₁₋₁₃-Isoparaffin, C₁₆₋₂₀-Isoparaffin, C₈₋₁₃-Isoparaffin, C₉₋₁₂-Isoparaffin, Limonen, Dioctylether oder Mischungen daraus.

Mit dem Begriff Waschbenzin wird eine Fraktion aus der Erdöldestillation mit einem Siedebereich von ca. 80-110 °C bezeichnet.

Limonen ist ein Naturstoff aus der Gruppe der Terpene (monocyclisches Monoterpen). Es gibt zwei Enantiomere, das (R)-(+)-Limonen (auch als D-(+)-Limonen bezeichnet) sowie das (S)-(-)-Limonen (auch als L-(-)-Limonen bezeichnet). Als flüssige, hydrophobe Verbindung können jeweils beide Enantiomere alleine oder das Racemat der beiden Enantiomere, welches auch als Dipenten bezeichnet wird, eingesetzt werden.

Limonen weist gute spreitende Eigenschaften auf und penetriert dadurch gut in textile Flächengebilde und den darauf befindlichen öl- und/oder fetthaltigen Verfärbungen. Außerdem weist Limonen einen angenehmen Geruch auf.

Dioctylether ist ein farbloses, geruchsneutrales Öl und besitzt einen hohen Spreitwert von 1600 mm²/ 10 min. Dioctylether ist beispielsweise unter der Bezeichnung Cetiol OE (ex Cognis) erhältlich.

Zusätzlich zu den vier essentiellen Bestandteilen kann das Textilbehandlungsmittel weitere Inhaltsstoffe enthalten, die die anwendungstechnischen und/oder ästhetischen Eigenschaften des Textilbehandlungsmittels weiter verbessern. Im Rahmen der vorliegenden Erfindung enthält das Textilbehandlungsmittel vorzugsweise zusätzlich einen oder mehrere Stoffe aus der Gruppe der anionischen Tenside, Gerüststoffe, Bleichmittel, Bleichkatalysatoren, Bleichaktivatoren, Enzyme, Verdickungsmittel, Elektrolyte, nichtwässrigen Lösungsmittel, pH-Stellmittel, Parfümzusammensetzungen, Parfümträger, Fluoreszenzmittel, Farbstoffe, Hydrotrope, Schauminhibitoren, Silikonöle, Soil-Release-Polymere, Vergrauungsinhibitoren, Einlaufverhinderer, Knitterschutzmittel, Farbübertragungsinhibitoren, weitere antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, weichmachenden Komponenten sowie UV-Absorber.

Besonders bevorzugte zusätzliche Inhaltsstoffe sind anionische Tenside, Gerüststoffe, Enzyme, Elektrolyte, nichtwässrigen Lösungsmittel, pH-Stellmittel, Parfümzusammensetzungen, Fluoreszenzmittel, Farbstoffe, Hydrotrope, Schauminhibitoren, Soil-Release-Polymere, Vergrauungsinhibitoren, Farbübertragungsinhibitoren, Verdickungsmittel, UV-Absorber sowie Mischungen daraus.

Die erfindungsgemäss verwendeten Textilbehandlungsmittel sind vorzugsweise flüssig und enthalten Wasser als Hauptlösungsmittel. Außerdem weist das Textilbehandlungsmittel einen sauren pH-Wert auf. Das Textilbehandlungsmittel kann als ein Waschmittel, ein Vorbehandlungsmittel oder als ein Waschmitteladditiv verwendet werden. Vorzugsweise ist das Textilbehandlungsmittel ein Waschmitteladditiv, welches zusammen mit einem Waschmittel im Hauptwaschgang eingesetzt wird, oder ein Vorbehandlungsmittel, welches gezielt, beispielsweise mittels Sprühens oder eines Roll-On-Applikators, auf die Deodorant-Flecken aufgebracht wird.

Die Herstellung eines erfindungsgemäß verwendeten Textilbehandlungsmittels erfolgt mittels üblicher und bekannter Methoden und Verfahren. So können beispielsweise die Bestandteile der Wasch-, Reinigungsmittel einfach in Rührkesseln vermischt werden, wobei Wasser, die sauren Komponenten, wie beispielsweise die Citronensäure, Ameisensäure, Phosphorsäure, usw., und die nichtionischen Tenside zweckmäßigerweise vorgelegt werden. Die nicht-wässrigen Lösungsmittel, falls vorhanden, werden vorzugsweise auch zu diesem Zeitpunkt zugegeben, die Zugabe kann aber auch zu einem späteren Zeitpunkt erfolgen. Dann werden die weiteren Bestandteile vorzugsweise portionsweise, hinzugefügt.

### Ausführungsbeispiele:

In Tabelle 1 sind die Zusammensetzungen von vier erfindungsgemäß verwendeten Textilbehandlungsmitteln E1 bis E4 gezeigt (alle Mengen sind in Gew.-% Aktivstoff, bezogen auf die Zusammensetzung, angegeben):

**Tabelle 1**

| | **E1** | **E2** | **E3** | **E4** |
|---|---|---|---|---|
| C₁₂₋₁₈-Fettalkohol mit 7 EO | ***--*** | ***--*** | ***--*** | 3,5 |
| C₈₋₁₀-Alkylpolyglycosid | 2 | 5 | 5 | -- |
| Citronensäure | 10 | 15 | 15 | 15 |
| Phosphorsäure | 1 | 3 | 2,5 | 2,5 |
| Ameisensäure | 0,5 | 1,5 | 1 | 1 |
| Zinkricinoleat | 0,1 | 1 | 0,5 | 0,5 |
| Limonen | -- | -- | -- | 1,5 |
| C₁₁₋₁₃-Isoparaffin | -- | -- | 2 | -- |
| Xanthan | -- | 0,35 | 0,8 | 0,8 |
| Parfüm | 0,3 | 0,3 | 0,3 | 0,3 |
| NaOH | 0,85 | 0,35 | 0,35 | 0,35 |
| Farbstoffe | -- | + | + | + |
| Wasser | Ad 100 | Ad 100 | Ad 100 | Ad 100 |
| pH-Wert | 2,05 | 1,5 | 1,5 | 1,5 |

Alle vier Textilbehandlungsmittel E1 bis E4 waren in der Lage, Verkrustungen und Verfärbungen, die aus der Anwendung von Deodorants resultieren, zu entfernen bzw. deutlich zu reduzieren. Außerdem zeigten Textilien, die mit diesen Textilbehandlungsmitteln behandelt wurden, bei ihrem Tragen, insbesondere im Bereich der Achseln, eine deutlich verminderte Bildung von Schlechtgerüchen. Die Textilbehandlungsmittel E3 und E4 zeigten eine besonders gute Wirkung gegen Verfärbungen, die durch die Anwendung von Deodorants entstanden sind.

Die erfindungsgemäß verwendeten Textilbehandlungsmittel weisen nicht nur eine Reinigungsleistung bei Verkrustungen, Verfärbungen und Schlechtgerüchen, die aus der Anwendung von Deodorants resultieren, sondern auch bei einer Vielzahl an weiteren Flecken/Anschmutzungen auf.

Die Bestimmung der Fähigkeit der Fleckentfernung des Textilbehandlungsmittels E1 erfolgte über die Bestimmung des Normfarbwertes Y (DIN 5033). Dazu wurde eine Haushaltswaschmaschine (Miele W 526) mit 3,5 kg Begleitwäsche sowie befleckten Stofflappen beladen. Zusätzlich wurden 90 ml eines flüssigen Vollwaschmittels zudosiert. In einem Fall wurden die befleckten Stofflappen mit dem Textilbehandlungsmittel E1 vorbehandelt, indem dieses aufgesprüht und 30 Minuten einwirken gelassen wurde. Die Bestimmung der Y-Werte erfolgte bei 420 nm (Gerät: Datacolor Spectraflash 600, 30 mm Blende).

**Tabelle 2: Remission: Y-Wert**

| Fleckart | Fleck ohne Vorbehandlung | Fleck mit Vorbehandlung mit E1 |
|---|---|---|
| Achselschweiß | 18,3 | 24,3 |
| Bratensauce | 62,2 | 74,0 |
| Kaffee | 64,4 | 69,6 |
| Blaubeeren | 64,2 | 72,2 |
| Schwarze Johannisbeeren | 51,5 | 56,1 |
| Brombeeren | 55,9 | 62,9 |
| Granatapfel | 79,4 | 87,7 |
| Rotwein (Bordeaux) | 70,0 | 79,3 |
| löslicher Tee | 45,5 | 73,5 |
| Früchtetee | 61,8 | 78,2 |
| Kräutertee | 56,0 | 85,9 |
| Schuhwichse | 51,9 | 61,7 |

Aus den Daten der Tabelle 2 wird deutlich, dass mit Hilfe des Textilbehandlungsmittels E1 eine Vielzahl an unterschiedlichen Flecken, insbesondere Flecken von Beeren, Kaffee, Tees und Rotwein, deutlich reduziert werden können.

## Patentansprüche

1. Verwendung eines Textilbehandlungsmittels mit einem pH-Wert im Bereich von 1,5 bis 4,5, umfassend
a) eine organische Säure,
b) eine anorganische Säure,
c) ein nichtionisches Tensid und
d) eine desodorierende Verbindung
beim Waschen, Reinigen und/oder Konditionieren von textilen Flächengebilden.

2. Verwendung eines Textilbehandlungsmittels gemäß Anspruch 1, **dadurch gekennzeichnet, dass** die organische Säure ausgewählt ist aus der Gruppe bestehend aus Citronensäure, Ameisensäure, Essigsäure, Weinsäure, Äpfelsäure, Oxalsäure, Milchsäure und Mischungen daraus.

3. Verwendung eines Textilbehandlungsmittels gemäß Anspruch 2, dass die organische Säure Citronensäure und/oder Ameisensäure umfasst.

4. Verwendung eines Textilbehandlungsmittels gemäß einem der Ansprüche 1 bis 3, **dadurch gekennzeichnet, dass** die anorganische Säure ausgewählt ist aus der Gruppe bestehend aus Phosphorsäure, Salzsäure und Mischungen daraus.

5. Verwendung eines Textilbehandlungsmittels gemäß einem der Ansprüche 1 bis 4, **dadurch gekennzeichnet, dass** die desodorierende Verbindung ausgewählt ist aus der Gruppe bestehend aus Zinkricinoleat, Zinkabietat, Cyclodextrin, ätherische Ölen, Triethylenglycol, Natriumhydrogencarbonat und Mischungen daraus.

6. Verwendung eines Textilbehandlungsmittels gemäß einem der Ansprüche 1 bis 5, **dadurch gekennzeichnet, dass** das Textilbehandlungsmittel zusätzlich eine flüssige, hydrophobe Verbindung enthält.

7. Verwendung eines Textilbehandlungsmittels gemäß Anspruch 6, dass die flüssige, hydrophobe Verbindung ausgewählt ist aus der Gruppe bestehend aus Waschbenzin, C₁₁₋₁₃-Isoparaffin, C₁₆₋₂₀-Isoparaffin, C₈₋₁₃-Isoparaffin, C₉₋₁₂-Isoparaffin, Limonen, Dioctylether und Mischungen daraus.

## Claims

1. The use of a textile treatment agent which has a pH in the range of from 1.5 to 4.5 and comprises
a) an organic acid,
b) an inorganic acid,
c) a non-ionic surfactant and
d) a deodorizing compound,
when washing, cleaning and/or conditioning textile fabrics.

2. The use of a textile treatment agent according to claim 1, **characterized in that** the organic acid is selected from the group consisting of citric acid, formic acid, acetic acid, tartaric acid, malic acid, oxalic acid, lactic acid and mixtures thereof.

3. The use of a textile treatment agent according to claim 2, **characterized in that** the organic acid comprises citric acid and/or formic acid.

4. The use of a textile treatment agent according to one of claims 1 to 3, **characterized in that** the inorganic acid is selected from the group consisting of phosphoric acid, hydrochloric acid and mixtures thereof.

5. The use of a textile treatment agent according to one of claims 1 to 4, **characterized in that** the deodorizing compound is selected from the group consisting of zinc ricinoleate, zinc abietate, cyclodextrin, essential oils, triethylene glycol, sodium hydrogen carbonate and mixtures thereof.

6. The use of a textile treatment agent according to one of claims 1 to 5, **characterized in that** the textile treatment agent additionally contains a liquid hydrophobic compound.

7. The use of a textile treatment agent according to claim 6, **characterized in that** the liquid hydrophobic compound is selected from the group consisting of petroleum benzine, C₁₁₋₁₃ isoparaffin, C₁₆₋₂₀ isoparaffin, C₈₋₁₃ isoparaffin, C₉₋₁₂ isoparaffin, limonene, dioctyl ether and mixtures thereof.

## Revendications

1. Utilisation d'un agent de traitement de textiles ayant une valeur de pH comprise dans une plage allant de 1,5 à 4,5, comprenant
a) un acide organique,
b) un acide inorganique,
c) un tensioactif non ionique et
d) un composé désodorisant
lors du lavage, nettoyage et/ou conditionnement de structures textiles plates.

2. Utilisation d'un agent de traitement de textiles selon la revendication 1, **caractérisée en ce que** l'acide organique est sélectionné dans le groupe constitué de l'acide citrique, l'acide formique, l'acide acétique, l'acide tartrique, l'acide malique, l'acide oxalique, l'acide lactique et de mélanges de ceux-ci.

3. Utilisation d'un agent de traitement de textiles selon la revendication 2, **caractérisée en ce que** l'acide organique comprend l'acide citrique et/ou l'acide formique.

4. Utilisation d'un agent de traitement de textiles selon une des revendications 1 à 3, **caractérisée en ce que** l'acide inorganique est sélectionné dans le groupe constitué de l'acide phosphorique, l'acide chlorhydrique et de mélanges de ceux-ci.

5. Utilisation d'un agent de traitement de textiles selon une des revendications 1 à 4, **caractérisée en ce que** le composé désodorisant est sélectionné dans le groupe constitué de ricinoléate de zinc, abiétate de zinc, cyclodextrine, huiles essentielles, triéthylène glycol, hydrogénocarbonate de sodium et de mélanges de ceux-ci.

6. Utilisation d'un agent de traitement de textiles selon une des revendications 1 à 5, **caractérisée en ce que** l'agent de traitement de textiles contient en plus un composé hydrophobe liquide.

7. Utilisation d'un agent de traitement de textiles selon la revendication 6, **caractérisée en ce que** le composé hydrophobe liquide est sélectionné dans le groupe constitué de distillat de pétrole, isoparaffine en C₁₁₋₁₃, isoparaffine en C₁₆₋₂₀, isoparaffine en C₈₋₁₃, isoparaffine en C₉₋₁₂, limonène, éther dioctylique et de mélanges de ceux-ci.
